# EUROPÄISCHE PATENTSCHRIFT

(11) **EP 3 683 594 B1**
(45) Veröffentlichungstag und Bekanntmachung des Hinweises auf die Patenterteilung: **06.09.2023**
(21) Anmeldenummer: 19152127.7
(22) Anmeldetag: 16.01.2019
(51) Int. Cl.: G01R 33/54

(54) **VERFAHREN ZUM BETRIEB EINER MAGNETRESONANZEINRICHTUNG, MAGNETRESONANZEINRICHTUNG, COMPUTERPROGRAMM UND ELEKTRONISCH LESBARER DATENTRÄGER**
MAGNETIC RESONANCE DEVICE, METHOD FOR OPERATING A MAGNETIC RESONANCE DEVICE, COMPUTER PROGRAM AND ELECTRONICALLY READABLE DATA CARRIER
PROCÉDÉ DE FONCTIONNEMENT D'UN DISPOSITIF DE RÉSONANCE MAGNÉTIQUE, DISPOSITIF DE RÉSONANCE MAGNÉTIQUE, PROGRAMME INFORMATIQUE ET SUPPORT DE DONNÉES ÉLECTRONIQUEMENT LISIBLE

(43) Veröffentlichungstag der Anmeldung: 22.07.2020
(73) Patentinhaber: Siemens Healthcare GmbH, 91052 Erlangen (DE)
(72) Erfinder: Greiser, Andreas, 91054 Erlangen (DE); Feiweier, Thorsten, 91099 Poxdorf (DE); Grodzki, David, 91058 Erlangen (DE); Kühn, Bernd, 91080 Uttenreuth (DE); Nittka, Mathias, 91083 Baiersdorf (DE); Paul, Dominik, 91088 Bubenreuth (DE); Speckner, Thorsten, 91058 Erlangen (DE)

(56) Entgegenhaltungen:
- DE-A1-102008 015 261
- DE-A1-102016 222 785
- BIELMEIER W, GREISER A, SPECKNER T, SCHNETTER V: "Optimierte Ausnutzung der maximalen Performanz der Leistungsverstärker einer MR-Anlage", PRIOR ART JOURNAL, Bd. 2015, Nr. 25, 17. Dezember 2015 (2015-12-17), Seiten 73-74, XP040672412, ISBN: 978-3-945188-28-6

## Beschreibung

Die Erfindung betrifft ein Verfahren zum Betrieb einer Magnetresonanzeinrichtung, die wenigstens eine Komponente mit einer Betriebsgrenze bezüglich eines Zustandsparameters aufweist, zur Aufnahme von Magnetresonanzdaten mit einem wenigstens eine Magnetresonanzsequenz umfassenden Protokoll, wobei das Protokoll durch Protokollparameter beschrieben ist und ein Protokollparametersatz vorliegt oder ermittelt wird, der bezüglich eines Protokollabschnitts eine beliebig häufige Wiederholung des Protokollabschnitts ohne Verletzung der Betriebsgrenze erlaubt. Daneben betrifft die Erfindung eine Magnetresonanzeinrichtung, ein Computerprogramm und einen elektronisch lesbaren Datenträger.

Die Magnetresonanzbildgebung ist als medizinische Modalität inzwischen etabliert. Dabei existieren eine Vielzahl möglichere Bildgebungstechniken, in denen im Rahmen von Protokollen eine oder mehrere unterschiedliche Magnetresonanzsequenzen genutzt werden können, um Magnetresonanzdaten eines Untersuchungsobjekts, insbesondere eines Patienten, aufzunehmen. Das bedeutet, Protokollparameter, die ein Protokoll beschreiben, umfassen insbesondere auch Sequenzparameter der darin verwendeten Magnetresonanzsequenzen.

Die Protokolle und Magnetresonanzsequenzen nutzen verschiedene Komponenten der Magnetresonanzeinrichtung, nachdem bei der Magnetresonanzbildgebung üblicherweise wenigstens ein Hochfrequenzpuls zur Anregung des Untersuchungsbereichs ausgestrahlt wird, wozu eine Hochfrequenzspulenanordnung eingesetzt werden kann, welche auch zum Empfang der entstehenden Magnetresonanzsignale eingesetzt werden kann, falls hierfür nicht eine spezielle Empfangsspulenanordnung, beispielsweise als Lokalspulenanordnung, vorgesehen ist. Die Leistung für auszusendende Hochfrequenzpulse wird üblicherweise mittels eines Hochfrequenzleistungsverstärkers (RFPA) erzeugt. Zur Ortskodierung werden in der Magnetresonanzbildgebung Gradienten eingesetzt, wobei die entsprechenden Gradientenfelder mittels einer entsprechenden Gradientenspulenanordnung erzeugt werden, die wiederum von einem Gradientenleistungsverstärker (GPA) gespeist wird. Weitere Komponenten der Magnetresonanzeinrichtung umfassen beispielsweise, neben dem selbstverständlich vorhandenen Grundfeldmagneten in der Hauptmagneteinheit, Shimspulenanordnungen, diverse Kühleinrichtungen und dergleichen.

Derartige Komponenten stellen Hardware dar, die aufgrund ihrer Auslegung Leistungsbeschränkungen bezüglich bestimmter Zustandsparameter aufweisen können. Beispielsweise werden bei gradientenlastigen Magnetresonanzsequenzen eine Vielzahl von Gradientenpulsen benötigt, welche für eine Erwärmung, insbesondere im Gradientenleistungsverstärker selbst, aber auch in anderen Komponenten der Magnetresonanzeinrichtung, führen können. Wird eine Betriebsgrenze für derart erwärmte Komponenten, beispielsweise den Gradientenleistungsverstärker, erreicht, muss die entsprechende Komponente abgeschaltet werden. Der Zustandsparameter ist in diesem Fall also die Temperatur, die einen die Betriebsgrenze beschreibenden Schwellwert nicht überschreiten darf. Daher sollen Magnetresonanzsequenzen oder eben allgemein Protokolle so ausgelegt werden, dass es möglichst nicht zu einer Überhitzung der Magnetresonanzeinrichtung kommt.

Magnetresonanzsequenzen, die eine hohe Gradientenleistung anfordern, sind beispielsweise sogenannte EPI-Sequenzen (Echo Planar Imaging), die beispielsweise im Rahmen der Diffusionsbildgebung häufig wiederholt ausgespielt werden können.

Bei TSE-Sequenzen (Turbo Spin Echo) tritt in einem anderen Beispiel das Problem auf, dass eine hohe und möglicherweise einschränkende Hochfrequenzaktivität erforderlich ist. Hier kann ein Zustandsparameter beispielsweise eine Ladung des Hochfrequenzleistungsverstärkers betreffen, so dass beispielsweise bei länger andauernden oder häufig schnell wiederholten TSE-Sequenzen Flipwinkel von Refokussierungspulsen reduziert werden können, um sicherzustellen, dass der Hochfrequenzleistungsverstärker nicht abgeschaltet werden muss.

Das Dokument DE 10 2008 015261 A1 beschreibt ein Verfahren zum Betrieb eines Magnetresonanzanlage. Ein Rechner ermittelt mindestens eine Gruppe von vorläufigen Ansteuersequenzen für Leistungsansteuereinrichtungen der Anlage derart, dass die Leistungsansteuereinrichtungen in der Lage sind, von ihnen angesteuerte bildbeeinflussende Emissionseinrichtungen der Anlage entsprechend den ermittelten vorläufigen Ansteuersequenzen anzusteuern und die Ansteuerung der Emissionseinrichtungen entsprechend den vorläufigen Ansteuersequenzen der jeweiligen Gruppe, soweit es die Ansteuerung der Emissionseinrichtungen betrifft, mit der durchzuführenden Messsequenz korrespondiert. Der Rechner ermittelt für jede Gruppe von vorläufigen Ansteuersequenzen unter Verwendung eines Modells zumindest der Emissionseinrichtungen und der Leistungsansteuereinrichtungen für diese Einrichtungen anhand eines jeweiligen Anfangsbelastungszustands und der jeweiligen Ansteuersequenz der Emissionseinrichtungen und der Leistungsansteuereinrichtungen jeweils einen Belastungszustandsverlauf. Der Rechner prüft für jede Gruppe, ob jeder für diese Gruppe ermittelte Belastungszustandsverlauf unterhalb einer Belastungsgrenze bleibt.

Das Dokument BIELMEIER W, GREISER A, SPECKNER T, SCHNETTER V: "Optimierte Ausnutzung der maximalen Performanz der Leistungsverstärker einer MR-Anlage" (PRIOR ART JOURNAL, Bd. 2015, Nr. 25, 17. Dezember 2015 (2015-12-17), Seiten 73-74, XP040672412, ISBN: 978-3-945188-28-6) offenbart einen Magnetresonanztomographen mit einem Monitor zur Vorhersage und Überwachung der abgegebenen Leistung der Verstärker, zum anderen eine übergeordnete Instanz zur Ablaufsteuerung der Messsequenzen. Der Monitor überwacht die Leistung der Verstärker mit einem Algorithmus, der die Belastung adäquat auf eine Kennzahl abbildet, die wiederum gegen eine Grenze verglichen werden kann. Der Algorithmus kann beispielsweise die Berechnung der Entladung der Energiespeicher über ein Ladungsbilanzmodell enthalten, die thermische Erwärmung der Endstufen messen bzw. mit einem thermischen Modell berechnen oder einen gleitenden zeitlichen Mittelwert, z.B. über sechs Minuten, über die abgegebene Leistung bilden. Mit der aktuellen Kennzahl ist eine Vorhersage möglich, ob die nächste Messsequenz lauffähig ist oder nicht. Die Ablaufsteuerung bedient sich dieser Vorhersage und kann so entscheiden, ob eine Messpause notwendig ist oder ob das Problem auch durch Umstellung der Reihenfolge der Messsequenzen umgangen werden kann, indem weniger performanzkritische Messungen vorgezogen werden.

Das Dokument DE 10 2016 222785 A1 betrifft ein automatisches Bestimmen von Parametern für eine Untersuchung mit einer Magnetresonanzanlage. Dabei wird eine Modellierung, welche die Parameter mit Relationen in Beziehung setzt, bestimmt. Jede Relation beschreibt eine mathematische Beziehung zwischen den Parametern der jeweiligen Relation. Dazu werden Werte oder Wertebereiche bestimmter der Parameter erfasst, welche von einem Benutzer der Magnetresonanzanlage vorgegeben werden. Die Parameter werden derart bestimmt, dass die Relationen eingehalten werden.

Mit anderen Worten wurde im Stand der Technik bereits vorgeschlagen, einstellbare Protokollparameter an der Magnetresonanzeinrichtung so stark einzuschränken, dass die Untersuchung, insbesondere ein bestimmtes Protokoll, durchgeführt werden kann, ohne dass Hardware-Komponenten zum Schutz abgeregelt beziehungsweise abgeschaltet werden müssen und ohne dass die Bildqualität aufgrund einer Unterschreitung der durch die Magnetresonanzsequenz angeforderten Leistungen beeinträchtigt wird. Aufgrund der komplexen Abhängigkeiten innerhalb der einzelnen Protokollparameter ist die Bestimmung von gerade noch möglichen, die Leistungsfähigkeit der Magnetresonanzeinrichtung vollständig ausnutzenden Werten der Protokollparameter oft nur durch das Ausrollen des vollständigen Messprotokolls beziehungsweise der darin enthaltenen Magnetresonanzsequenzen möglich, bei mehreren Protokollen einer Untersuchung gegebenenfalls auch nur durch Ausrollen des gesamten Untersuchungsverlaufs.

In diesem Kontext wurde es im Stand der Technik bereits vorgeschlagen, den Einfluss von Protokollen auf den Systemzustand bezüglich des Zustandsparameters zu modellieren, beispielsweise über ein Zustandsparametermodell. Die Idee hierbei ist es, Magnetresonanzsequenzen innerhalb eines derartigen Zustandsparametermodells auszurollen, um ihren Effekt auf den Zustandsparameter zu simulieren. So ist es möglich, abzuschätzen, wie lange die Magnetresonanzeinrichtung bei einer bestimmten Leistung betrieben werden kann, bevor die Betriebsgrenze erreicht ist. Ein anschauliches Beispiel hierfür betrifft die Erwärmung. Kühleinrichtungen der Magnetresonanzeinrichtung können nur eine endliche Wärmemenge abführen. Wird durch Beanspruchung des Gradientenleistungsverstärkers oder des Hochfrequenzleistungsverstärkers oder anderer Komponenten mehr Wärme zugeführt, als die Kühleinrichtung abführen kann, heizt sich die Magnetresonanzeinrichtung auf und muss bei Erreichen einer Betriebsgrenze, beispielsweise eines Schwellwerts für die Temperatur, abgeschaltet werden. So kann über eine einfache Betrachtung des Temperaturzustands der Magnetresonanzeinrichtung eine Vielzahl von zur Performance der Magnetresonanzeinrichtung beitragenden Aspekten zusammengefasst werden.

Um die Komplexität, insbesondere auch hinsichtlich der Bedienung der Magnetresonanzeinrichtung, zu verringern, um mithin die Verwendung der Magnetresonanzeinrichtung einfach und zuverlässig zu gestalten, wird bei im Stand der Technik bekannten Magnetresonanzeinrichtungen das Prinzip der beliebigen Wiederholbarkeit von Messprotokollen verfolgt, was auch unter den Begriff "run once, run ever"-RORE bekannt ist. Das bedeutet, Magnetresonanzsequenzen werden bezüglich der Protokollparameter grundsätzlich so ausgelegt, dass zum einen jede Messung beliebig oft durchgeführt werden kann und zum anderen nicht vom Anfangssystemzustand der Magnetresonanzeinrichtung abhängt. Selbst bei einer aufgeheizten Magnetresonanzeinrichtung kann jedes RORE-Protokoll durchgeführt werden, da es nicht zu einer weiteren Netto-Aufheizung führt, die zum Erreichen der Betriebsgrenze führen könnte.

In einem anderen Ansatz werden Protokolle so bestimmt, dass sie nur ein einziges Mal durchgeführt werden können ("run once", RO). Derartig bestimmte Protokollparametersätze nutzen mithin die Leistungsfähigkeit der Magnetresonanzeinrichtung soweit aus, dass sich die Zustandsparameter bis zur Betriebsgrenze hin verändern dürfen. Damit einher geht die Notwendigkeit, den Anfangssystemzustand der Magnetresonanzeinrichtung in die Modellierung mit einzubeziehen.

Ist die Magnetresonanzeinrichtung beispielsweise schon aufgeheizt, kann keine Messung mehr durchgeführt werden, die die Magnetresonanzeinrichtung beziehungsweise die konkret betroffenen Komponenten über die Betriebsgrenze hinaus aufheizen würde. Da sich die Magnetresonanzeinrichtung im Ruhezustand wieder abkühlt, kann für ein gegebenes RO-Protokoll ermittelt werden, ab wann es wieder durchgeführt werden kann. Die Verwendung von RO-Protokollen kann also durch die Einführung von Messpausen unterstützt werden, was aber den Messablauf unter Umständen signifikant beeinträchtigen kann. Daher wurden derartige Ansätze bislang nicht weiter verfolgt.

Bisher wurden die Werte der Protokollparameter, die die Magnetresonanzeinrichtung für eine Messung zulässt, so konservativ eingeschränkt, dass die Leistungsfähigkeit der Magnetresonanzeinrichtung im Allgemeinen nicht vollständig ausgenutzt werden kann. Insbesondere kann vorgesehen sein, dass lediglich Protokolle gemäß dem RORE-Ansatz eingesetzt werden. Dies führt zu einer suboptimalen Ausnutzung der verfügbaren Systemperformance, was insbesondere vor dem Hintergrund eines steigenden Kostendrucks für die verwendeten Hardware-Komponenten fragwürdig ist. Ferner wurden bereits Magnetresonanzeinrichtungen vorgeschlagen, die zu relativ hohen Spitzenleistungen in der Lage sind, jedoch damit einhergehend eine geringere nominale Dauerleistung bereitstellen können, was die erreichbare Magnetresonanzdatenqualität bei RORE-Protokollen weiter reduzieren beziehungsweise die Aufnahmezeit weiter verlängern kann, obwohl die Magnetresonanzeinrichtung grundsätzlich zu deutlich besseren Leistungen im Stande wäre.

Der Erfindung liegt daher die Aufgabe zugrunde, eine Möglichkeit zur verbesserten Ausnutzung der Leistungsfähigkeit einer Magnetresonanzeinrichtung bei entsprechendem nutzerseitigem Bedarf anzugeben.

Diese Aufgabe wird gelöst durch ein Verfahren, eine Magnetresonanzeinrichtung, ein Computerprogramm und einen elektronisch lesbaren Datenträger gemäß den unabhängigen Ansprüchen. Vorteilhafte Ausgestaltungen ergeben sich aus den Unteransprüchen.

Bei einem Verfahren der eingangs genannten Art sind erfindungsgemäß folgende Schritte vorgesehen:
- Empfangen eines eine erhöhte Ausnutzung der Leistungsfähigkeit der Magnetresonanzeinrichtung bei Begrenzung der Wiederholbarkeit des Protokollabschnitts beschreibenden, benutzerseitig gewählten Boostparameters von einer Benutzerschnittstelle,
- zumindest für den Protokollabschnitt, wenigstens teilweise automatisches Ermitteln von Anpassungsparametern, die wenigstens einen Teil der Protokollparameter umfassen, unter Berücksichtigung des Boostparameters derart, dass die durch den Boostparameter beschriebene Wunschanzahl an Wiederholungen bei Einhaltung der Betriebsgrenze gegeben ist, mittels eines Prozessors, und
- Aufnahme von Magnetresonanzdaten mit dem Protokoll unter Verwendung der ermittelten Anpassungsparameter mittels eines Controllers,
wobei die Ermittlung der Anpassungsparameter unter Verwendung einer benutzerseitig wählbaren Lösungsstrategie erfolgt, und die Lösungsstrategie eine Zielvorgabe beschreibt, gewählt aus der Gruppe umfassend eine Reduzierung der Gesamtzeit zur Durchführung des Protokolls, Erhöhung der Magnetresonanzdatenqualität und Maximierung oder Minimierung wenigstens eines bestimmten Protokollparameters.

Es wird mithin ein computerimplementiertes Verfahren vorgeschlagen, welches einen benutzerseitig vorgebbaren Boostparameter nutzt, um gegenüber dem Konzept der beliebig häufigen, also unbegrenzten, Wiederholbarkeit eines Protokolls bzw. Protokollabschnitts eine höhere Leistungsfähigkeit für eine bestimmte Messung verfügbar zu machen. Hierbei kann ein Protokollabschnitt grundsätzlich auch das gesamte Protokoll umfassen, häufiger aber wird der Protokollabschnitt eine besonders hohe Anforderung stellende Magnetresonanzsequenz beziehungsweise den betreffenden Protokollanteil umfassen, insbesondere also sogenannte High-Performance-Sequenzen, im Fall der Diffusionsbildgebung beispielsweise EPI-Sequenzen, nachdem andere in einem derartigen Protokoll verwendete Magnetresonanzsequenzen zu einer deutlich geringeren Gradientennutzung und somit einer deutlich geringeren Erwärmung von Komponenten der Magnetresonanzeinrichtung führen.

Es wird mithin vorgeschlagen, von der strikten RORE-Anforderung an durchzuführende Protokolle abzuweichen und auf benutzerseitige Anforderung eine gewisse Aufheizung der Magnetresonanzeinrichtung beziehungsweise eine gewisse sonstige Änderung eines Zustandsparameters im Hinblick auf eine Systemgrenze zuzulassen. Einem durchzuführenden Protokoll wird dabei vom Benutzer ein Boostparameter zugeordnet, der festlegt, wie oft das betreffende Protokoll gemessen werden können soll, bevor die Betriebsgrenze erreicht wird. Dabei kann in einer besonders vorteilhaften und intuitiven Ausführungsform vorgesehen sein, dass der Boostparameter unmittelbar die Wunschanzahl beschreibend empfangen wird. Die Grundeinstellung, die zu einem RORE-Protokoll führt, wäre in diesem Fall ein Boostparameter von ∞ (unendlich), während ein Abrufen der maximalen Leistungsfähigkeit der Magnetresonanzeinrichtung bei einem Boostparameter von 1, also entsprechend einem RO-Protokoll, gegeben wäre.

Durch benutzerseitige Vorgabe des Boostparameters kann der Benutzer mit der Zeit lernen, was an seiner Magnetresonanzeinrichtung und bei unterschiedlichen gegebenen Untersuchungszielen an Leistungsgewinn möglich ist, insbesondere dann, wenn zwischen den Untersuchungen unterschiedlicher Patienten Untersuchungspausen liegen, innerhalb welcher sich die Zustandsparameter, insbesondere, wie beschrieben, die Temperatur, wieder im Wesentlichen auf ein Grundniveau zurückbewegen können.

Ein äußerst intuitives Beispiel ist dabei durch die Diffusionsbildgebung gegeben, bei der Protokolle beziehungsweise Protokollabschnitte mit einer EPI-Sequenz als Magnetresonanzsequenz ggf. häufig wiederholt werden müssen, so dass eine gewisse Wiederholbarkeit durchaus wünschenswert ist. Letztendlich kann der Boostparameter dann zweckmäßigerweise so gewählt werden, dass die Wunschanzahl der maximal notwendigen Anzahl der Messungen des Protokollabschnitts im gesamten Workflow einer Untersuchung entspricht. Dies gilt jedenfalls dann, worauf im Folgenden noch näher eingegangen werden wird, wenn sonstige Protokollanteile neben dem Protokollabschnitt die RORE-Bedingung erfüllen, mithin dort eine unbegrenzte Wiederholbarkeit vorliegt.

Dies ist, wie bereits beschrieben, üblicherweise dann gegeben, wenn der Protokollabschnitt wenigstens die die stärksten Auswirkungen auf den Zustandsparameter, insbesondere die Temperatur, aufweisende Magnetresonanzsequenz enthält. Bei der Diffusionsbildgebung betrifft dies beispielsweise Protokollanteile, in denen eine EPI-Sequenz eingesetzt wird, nachdem andere, im Rahmen des Protokolls beziehungsweise allgemein der Untersuchung eingesetzte Sequenzen, beispielsweise HASTE-Sequenzen oder MPRAGE-Sequenzen, eine deutlich geringere Gradientennutzung benötigen, beispielsweise lediglich 5 % einer EPI-Sequenz, so dass eine High-Performance-Sequenz sozusagen stellverstretend für das gesamte Protokoll herangezogen werden kann. Dies reduziert im Übrigen auch den Aufwand bei der Ermittlung der Anpassungsparameter, nachdem dann insbesondere nicht das gesamte Protokoll ausgerollt werden muss, sondern es ausreichend ist, den starken Anteil der High-Performance-Sequenz zu betrachten, nachdem die Abschätzung ohnehin ein "Worst-Case-Szenario" betrifft, da in der Realität Messpausen und dergleichen existieren, in denen der Zustandsparameter entspannen kann.

Damit erlaubt die vorliegende Erfindung eine verbesserte Ausnutzung der verfügbaren Hardware-Leistungsfähigkeit in Kombination mit der Vermeidung von komplexen Abhängigkeiten über den gesamten Untersuchungsablauf, wie sie sich aus einer protokollübergreifenden Simulation der Leistungsanforderung im Wechselspiel mit Abkühlphasen und dergleichen ergeben würden. Nachdem mit besonderem Vorteil nur ein einziger Boostparameter verwendet wird und benutzerseitig einstellbar ist, ist eine für den Benutzer einfache, intuitive Umsetzung gegeben, die auch hier die Komplexität nicht erhöht.

Die Anpassungsparameter, die letztlich bestimmte, ausgewählte Protokollparameter sind, welche den Protokollabschnitt betreffen, lassen sich dabei durch relativ einfache Überlegungen ermitteln. Selbstverständlich müssen sie zunächst der Einstellbarkeit zugänglich sein. Die Anpassungsparameter können durch Identifizieren geeigneter Modifikationen des Protokollabschnitts ermittelt werden. Beispielsweise können als Anpassungsparameter Zeitablaufsparameter, insbesondere Echozeiten (TE) und/oder Repetitionszeiten (TR) betrachtet werden, um beispielsweise Abkühlungsphasen zu reduzieren beziehungsweise zur Verfügung zu stellen. Andere Parameter, die betrachtet werden können, betreffen beispielsweise den Flipwinkel, Gradientenstärken und dergleichen, wobei auch Zeitabfolgen veränderbar sein können. Ersichtlich existieren eine Vielzahl von Möglichkeiten, Anpassungsparameter zu wählen, welche insbesondere auch von dem gewünschten Effekt der verbesserten Leistungsfähigkeitsausnutzung abhängen können.

Konkret kann vorgesehen sein, dass zur Ermittlung der Anpassungsparameter das Verhalten des wenigstens einen Zustandsparameters, insbesondere durch Ausrollen des Protokollabschnitts und/oder unter Verwendung eines Zustandsparametermodells, simuliert wird. Insbesondere kann also vorgesehen sein, die Protokollereignisse auszurollen, um den Einfluss auf den wenigstens einen betrachteten Zustandsparameter, insbesondere die Temperatur, abzubilden. Dabei werden bevorzugt heuristische, durch einen Prozessor möglichst einfach umsetzbare Modellierungen angestrebt, beispielsweise lineare Zustandsparametermodelle und/oder sonstige, wenige komplexe Zustandsparametermodelle. Beispielsweise kann in einem konkreten Fall vorgesehen sein, für ein lineares Temperaturmodell der Magnetresonanzeinrichtung die durchschnittliche Gradientenaktivität über ein gleitendes Fenster, beispielsweise einer Länge von sechs Minuten zu betrachten. Insgesamt sind im Stand der Technik jedoch bereits eine Vielzahl von Möglichkeiten vorgeschlagen worden, die Auswirkungen von Protokollen und Magnetresonanzsequenzen auf den Systemzustand der Magnetresonanzeinrichtung zu modellieren und nachzuverfolgen, die selbstverständlich insgesamt auch im Rahmen der vorliegenden Erfindung eingesetzt werden können, um den Einfluss des Protokollabschnitts auf die Zustandsparameter abschätzen zu können.

Die Ermittlung der Anpassungsparameter erfolgt unter Verwendung einer benutzerseitig wählbaren Lösungsstrategie und vorzugsweise eines Optimierungsverfahrens. Ein Optimierungsverfahren kann beispielsweise darauf abzielen, dass bei der Wunschanzahl an Wiederholungen des Protokollabschnitts unter Nutzung der Anpassungsparameter die Leistungsfähigkeit der Magnetresonanzeinrichtung soweit ausgenutzt wird, dass möglichst dicht an die Betriebsgrenze herangekommen wird, wobei hier auch ein gewisser Sicherheitsabstand zur Betriebsgrenze vorgegeben und entsprechend eingehalten werden kann. Dann kann beispielsweise vorgesehen sein, das Zustandsparametermodell zu nutzen, um festzustellen, inwieweit eine Veränderung des Zustandsparameters hin zur Betriebsgrenze bei aktuellen Testwerten für die Anpassungsparameter stattfindet, woraufhin dann die Testwerte angepasst werden können, bis, wie bei Optimierungsverfahren üblich, ein Abbruchkriterium erreicht ist. Dabei können grundsätzlich im Stand der Technik bekannte Optimierungsalgorithmen auch im Rahmen der vorliegenden Erfindung eingesetzt werden.

Nachdem, wie bereits dargelegt wurde, komplexe Abhängigkeiten zwischen den verschiedenen Protokollparametern/Anpassungsparametern beziehungsweise Zustandsparametern existieren können und zudem die weitergehende Ausnutzung der Leistungsfähigkeit in unterschiedlicher Hinsicht genutzt werden kann, werden im Rahmen der vorliegenden Erfindung bestimmte Lösungsstrategien verwendet um insbesondere optimale Anpassungsparameter auffinden zu können. In diesem Zusammenhang ist vorgesehen, dass die Lösungsstrategie eine Zielvorgabe beschreibt, gewählt aus der Gruppe umfassend eine Reduzierung der Gesamtzeit zur Durchführung des Protokolls, Erhöhung der Magnetresonanzdatenqualität und Maximimierung oder Minimierung wenigstens eines bestimmten Protokollparameters. Beispielsweise wurde im Stand der Technik bereits vorgeschlagen, zusätzliche Messpausen einzuführen, um eine Abkühlung von Komponenten der Magnetresonanzeinrichtung zu erlauben, so dass sich beispielsweise Repetitionszeiten beziehungsweise die Gesamtuntersuchungsdauer verlängert haben. Zur Verkürzung der Dauer der Messung mit dem Protokoll kann beispielsweise darauf abgezielt werden, Repetitionszeiten zu minimieren. Andere Aspekte können beispielsweise auf eine höhere Bildqualität abzielen, beispielsweise durch Einsatz höherer Flipwinkel, stärkerer Gradienten und dergleichen. Letztlich kann die Lösungsstrategie also insbesondere definieren, welche Anpassungsparameter, gegebenenfalls mit welcher Priorität, minimiert beziehungsweise maximiert werden. Dabei sind durchaus auch gemischte Ansätze denkbar, bei denen unterschiedliche Optimierungsziele mit unterschiedlichen Gewichtungen verfolgt werden. Beispielhafte Lösungsstrategien können eine Minimierung der Repetitionszeit (TR), eine Minimierung der Echozeit (TE) beziehungsweise eine Minimierung der Repetitionszeit, danach der Echozeit, umfassen.

In weiterer Ausgestaltung der vorliegenden Erfindung kann auch vorgesehen sein, dass die Lösungsstrategie eine Vorgabe von benutzerseitig einstellbaren Wertebereichen für wenigstens einen Anpassungsparameter umfasst. Beispielsweise können im oben genannten Beispiel maximale Werte für die Echozeit und die Repetitionszeit vorgegeben werden. So können benutzerseitig ungewollte, extreme Lösungen im Lösungsraum vermieden werden. Dabei sei an dieser Stelle angemerkt, dass die Lösungsstrategie auch abschließend oder nicht abschließend Anpassungsparameter unmittelbar definieren kann. Beispielsweise kann eine Lösungsstrategie von vornherein vorgeben, den Performancegewinn allein in eine Minimierung von einer Echozeit oder einer Repetitionszeit zu investieren, oder aber in eine Minimierung beider Parameter. Insbesondere bei mehreren derart ausgewählten, zu optimierenden Anpassungsparametern ist die Einschränkung des zulässigen Wertebereichs, gerade im Hinblick auf Maximalwerte bei Echozeit und Repetitionszeit, besonders zweckmäßig.

Gerade im Fall der benutzerseitigen Vorgabe von Wertebereichen kann es auch vorkommen, dass durch Anwendung der Lösungsstrategie keine abschließende Lösung gefunden werden kann, beispielsweise auch, was noch genauer diskutiert werden wird, wenn der aktuelle Systemzustand der Magnetresonanzeinrichtung bezüglich des Zustandsparameters zu Beginn des Protokolls beziehungsweise des Protokollabschnitts zu ungünstig ist. In einem solchen Fall sieht eine vorteilhafte Ausgestaltung der Erfindung vor, dass bei einer durch einen Konflikt, insbesondere einen ungeeigneten Anfangssystemzustand und/oder einen nicht einhaltbaren, benutzerseitig vorgegebenen Wertebereich für einen Anpassungsparameter, gegebenen Nichtermittelbarkeit der Anpassungsparameter derart, dass die durch den Boostparameter beschriebene Wunschanzahl an Wiederholungen bei Einhaltung der Betriebsgrenze gegeben ist, als Konfliktstrategie wenigstens ein, insbesondere weiterer, Protokollparameter und/oder der Boostparameter zur Lösung des Konflikts angepasst wird und/oder die Lösungsstrategie geändert wird. Hierbei kann konkret vorgesehen sein, dass die Konfliktstrategie vorgegeben wird und/oder von einem Benutzer über das Benutzerinterface abgefragt wird.

Dabei sei an dieser Stelle nochmals angemerkt, dass die vorliegende Erfindung darauf abzielt, die Bedienung für den Benutzer möglichst wenig zu verkomplizieren. Im einfachsten Fall muss der Benutzer lediglich den, vorzugsweise genau einen, Boostparameter vorgeben. Die Lösungsstrategie, welche optional einstellbar ist, kann beispielsweise definieren, durch welche Anpassungsparameter oder Kombinationen von Anpassungsparametern die potentielle Leistungsbeschränkung aufgelöst werden soll. Im Fall mehrerer Anpassungsparameter sieht eine zweckmäßige Weiterbildung der Erfindung, wie bereits erwähnt, vor, dass der Wertebereich für die Anpassungsparameter benutzerseitig einschränkbar ist, beispielsweise durch Angabe eines oberen oder unteren Grenzwerts, je nach Anpassungsparameter. Dabei ist selbstverständlich darauf hinzuweisen, dass bereits grundsätzlich vorliegende Einschränkungen seitens der Magnetresonanzeinrichtung beziehungsweise messtechnische Einschränkungen berücksichtigt werden, so dass beispielsweise beliebig kurze Echozeiten oder dergleichen ohnehin nicht möglich sind. Nach Vorgabe wenigstens des, vorzugsweise genau einen, Boostparameters erfolgt eine dem Boostparameter und der Lösungsstrategie entsprechende interne Umparametrierung des Protokolls, so dass in den meisten Fällen keine weitere Benutzerinteraktion notwendig ist und ein optimiertes Protokoll im Hintergrund generiert werden kann. Nur in dem Fall, in dem ausgewählte Werte keine konsistente Lösung für die Anpassungsparameter liefern, kann, beispielsweise in Form eines zusätzlichen Pop-up-Fensters, eine Konfliktstrategie zur Auflösung des Konflikts abgefragt werden.

Dabei ist es grundsätzlich denkbar, zur Vereinfachung des hier beschriebenen Konzeptes davon auszugehen, dass zwischen Untersuchungen die Zusatzparameter sich bestimmten Ausgangswerten zumindest wieder annähern, so dass ein festgelegter Startsystemzustand der Magnetresonanzeinrichtung bezüglich der Zustandsparameter abgenommen werden kann. Beispielsweise kann davon ausgegangen werden, dass zwischen zwei Untersuchungsvorgängen unterschiedlicher Patienten eine hinreichende Abkühlung der Magnetresonanzeinrichtung erfolgt, während die Patienten wechseln bzw. der neue Patient positioniert wird.

In einer bevorzugten Ausgestaltung der vorliegenden Erfindung ist jedoch vorgesehen, dass bei der Ermittlung der Anpassungsparameter ein aktueller Systemzustand der Magnetresonanzeinrichtung bezüglich des Zustandsparameters vor Beginn des Protokollabschnitts, insbesondere ein aktueller Wert des Zustandsparameters vor Beginn des Protokollabschnitts, berücksichtigt wird. Insbesondere kann auf diese Weise auch berücksichtigt werden, dass der Austrag von beispielsweise Wärme aus der Magnetresonanzeinrichtung von der absoluten aktuellen Temperatur abhängt, nachdem, je höher das Temperaturgefälle der Komponenten gegen das Kühlmedium der Kühleinrichtung ist, desto mehr Wärme von den Komponenten abgegeben werden kann. Somit ist die Berücksichtigung eines Anfangszustandsparameters für die Ermittlung der Anpassungsparameter von Vorteil. Dabei wird der aktuelle Systemzustand bevorzugt auch zwischen Protokollen beziehungsweise außerhalb des Protokollabschnitts mitgeführt beziehungsweise für den Beginn des Protokollabschnitts prädiziert. Er kann aus Messwerten für den Zustandsparameter und/oder durch Nutzung des und/oder eines weiteren Zustandsparametermodells ermittelt werden. Es ist mithin beispielsweise möglich, eine Temperatur der Magnetresonanzeinrichtung und/oder wenigstens einer Komponente über eine geeignete Modellierung der Erwärmung auf Basis der Protokollabfolgen zu ermitteln, beispielsweise zentral in der Steuereinrichtung der Magnetresonanzeinrichtung. Möglich ist es zusätzlich beziehungsweise alternativ auch, tatsächlich gemessene Temperaturwerte aus vorhandenen Temperatursensoren an wenigstens einer erwärmten Komponente zu ermitteln und zu verwenden. Dabei müssen nicht zwangsläufig wenig Einfluss auf den Zustandsparameter nehmende, vor Beginn des Protokollabschnitts zu messende Protokollanteile berücksichtigt werden, insbesondere durch Ausrollen, sondern in solchen Fällen kann auch von dem aktuellen Systemzustand der Magnetresonanzeinrichtung als weiterhin aktueller Systemzustand zu Beginn des Protokollabschnitts ausgegangen werden.

Sollte es sich doch einmal ergeben, dass der Protokollabschnitt häufiger wiederholt werden muss als ursprünglich geplant und somit der Wunschanzahl entsprechend, so muss dies nicht zwangsläufig einen Abbruch bei unvollständiger Messung bedeuten. Vielmehr sieht eine vorteilhafte Weiterbildung der vorliegenden Erfindung vor, dass bei einer benutzerseitig angeforderten und/oder automatisiert ausgelösten, die Wunschanzahl überschreitenden Anzahl von Wiederholungen des Protokollabschnitts für die Wiederholungen nach der Wunschanzahl der Protokollparametersatz für unbegrenzte Wiederholungen verwendet wird und/oder eine Messpause eingefügt wird. Falls also wider Erwarten der Protokollabschnitt öfter als die Wunschanzahl wiederholt werden muss, beispielsweise aufgrund von schlechter Bildqualität wegen Patientenbewegung, kann unmittelbar ein äquivalentes Protokoll mit RORE-Eigenschaften (und damit einhergehender geringerer Performance) eingesetzt werden. Alternativ könnte durch eine Messpause wieder ein akzeptabler Wert des wenigstens einen Zustandsparameters erreicht werden, beispielsweise also die Magnetresonanzeinrichtung soweit heruntergekühlt werden, dass eine weitere Messung des Protokolls mit den ermittelten Anpassungsparametern ermöglicht wird.

Im Rahmen der vorliegenden Erfindung kann grundsätzlich vorgesehen sein, dass in außerhalb des Protokollabschnitts gelegenen Protokollanteilen des Protokolls lediglich beliebig oft wiederholbare Magnetresonanzsequenzen verwendet werden. Das bedeutet, es kann eine Grundannahme getroffen werden, dass in einem Workflow nur ein Protokollabschnitt mit höherer Leistungsanforderung verwendet werden soll, während alle anderen Protokollanteile beziehungsweise Magnetresonanzsequenzen der RORE-Anforderung genügen können.

Jedoch ist es im Rahmen der vorliegenden Erfindung vorteilhaft auch denkbar, in dem Protokoll mehrere Protokollabschnitte zu definieren, denen jeweils Boostparameter zugeordnet werden. Hierbei kann konkret vorgesehen sein, dass bei mehreren Protokollabschnitten der Einfluss des zeitlich früher durchgeführten Protokollabschnitts bei der Wunschanzahl an Wiederholungen auf den Anfangssystemzustand der Magnetresonanzeinrichtung bezüglich des Zustandsparameters bei Beginn des seitlich nächsten Protokollabschnitts berücksichtigt wird. Hierbei kann im Übrigen auch berücksichtigt werden, dass sich die Magnetresonanzeinrichtung zwischen unterschiedlichen Protokollabschnitten wieder abkühlen kann, wenn zwischen diesen Protokollabschnitten beispielsweise RORE-Protokollanteile vermessen werden beziehungsweise Messpausen liegen. Dabei können insbesondere Protokollanteile innerhalb eines Workflows benutzerseitig oder automatisiert auch geschickt platziert werden, so dass beispielsweise bei zwei Protokollabschnitten, für die eine höhere Leistungsfähigkeit genutzt werden soll, einer dieser Protokollabschnitte am Anfang des Gesamtprotokolls und einer der Protokollabschnitte am Ende des Protokolls platziert werden kann, so dass dazwischen eine hinreichende Abkühlung beziehungsweise allgemein Rücksetzung des Zustandsparameters erfolgen kann.

Es sei dennoch darauf hingewiesen, dass in den meisten Fällen Workflows beziehungsweise Protokolle, die einen Untersuchungsvorgang eines Patienten beschreiben, wie eingangs angedeutet, nur eine Art von äußerst leistungsintensiven Protokollabschnitten enthalten, so dass der typische Anwendungsfall der vorliegenden Erfindung die Abfolge von Protokollabschnitten in einem kompakten Zeitabschnitt im Workflow betrifft, beispielsweise Protokollabschnitte mit EPI-Sequenzen bei einer Ganzkörper-Diffusionsmessung.

Eine besonders vorteilhafte Weiterbildung der vorliegenden Erfindung sieht vor, dass bei einem durch eine Überwachungseinheit der Magnetresonanzeinrichtung aufgrund der Überschreitung einer Betriebsgrenze unterbrochenen Protokoll dieses mit dem Protokollparametersatz für unbegrenzte Wiederholungen fortgesetzt oder neu gestartet wird. Üblicherweise weisen Magnetresonanzeinrichtungen Überwachungseinheiten auf, die auch Teil der Steuereinrichtung der Magnetresonanzeinrichtung sein können und dann, wenn die Betriebsgrenze erreicht ist, einen Nothalt durchführen und eine aktuell laufende Messung abbrechen können. Dann kann besonders vorteilhaft vorgesehen sein, dass das Protokoll mit dem Protokollparametersatz für unbegrenzte, also beliebig häufige, Wiederholungen fortgesetzt wird. Denn dieser ist so bestimmt, dass selbst bei bereits nahe an der Betriebsgrenze betriebener Magnetresonanzeinrichtung diese Betriebsgrenze gerade nicht überschritten wird. Jedoch werden dennoch äquivalente Magnetresonanzdaten geliefert, nur lediglich mit längerer Zeitdauer beziehungsweise reduzierter Qualität der Magnetresonanzdaten. Nichtsdestotrotz wird die Messung dann dennoch vervollständigt. Möglich ist es in einer Alternative auch, das Protokoll mit dem RORE-Protokollparametersatz neu zu starten, was jedoch bevorzugt erst nach einer entsprechenden Abfrage vom Benutzer erfolgt. In allen hier beschriebenen Fällen ist es jedoch zweckmäßig, den Benutzer über eine entsprechende Ausgabeeinrichtung über den Zwischenfall zu informieren. Insbesondere dann, wenn das Protokoll mit verringerter Ausnutzung der Leistungsfähigkeit anhand des RORE-Protokollparametersatzes fortgeführt wird, wird der Einfluss des Messabbruchs gering gehalten, nachdem die bisher gemessenen Magnetresonanzdaten genutzt werden können und nur durch die restliche Messung im RORE-Modus ergänzt werden. Der bekannte, vorliegende Protokollparametersatz für unbegrenzte Wiederholungen bietet mithin eine Rückfallebene, die in diesem Zusammenhang besonders vorteilhaft genutzt werden kann. So ist es beispielsweise denkbar, wenn einige Schichten des Untersuchungsbereichs bereits vollständig gemessen wurden, die restlichen Schichten beispielsweise mit einem niedrigeren Flipwinkel oder einem schlechteren Schichtprofil beziehungsweise mit längerer Repetitionszeit oder Echozeit zu akquirieren.

Beispielsweise kann der Zustandsparameter eine Temperatur der Komponente sein, wie bereits dargelegt wurde. Dies gilt insbesondere im Hinblick auf gradientenintensive Magnetresonanzsequenzen. Jedoch sind auch andere Zustandsparameter denkbar, beispielsweise ein Ladezustand in einer Leistungsverstärkereinrichtung, insbesondere einer Hochfrequenzleistungsverstärkereinrichtung, als Komponente. Der Protokollabschnitt kann eine Messung mit einer EPI-Magnetresonanzsequenz und/oder eine Diffusionsmessung und/oder eine Messung mit einer TSE-Sequenz umfassen.

Ein besonders vorteilhaftes Ausführungsbeispiel der vorliegenden Erfindung ergibt sich, wenn eine Anwendung auf Diffusionsmessungen, insbesondere Ganzkörperdiffusionsmessungen, mit EPI-Magnetresonanzsequenzen erfolgt. Dann wird als Zustandsparameter bevorzugt eine Temperatur, insbesondere eine Temperatur der Gradientenspulenanordnung und/oder der Gradientenleistungsverstärkereinrichtung betrachtet, wobei es sich auch als zweckmäßig erwiesen hat, eine allgemeine Temperatur für die Magnetresonanzeinrichtung zu definieren und, beispielsweise in einem Zustandsparametermodell, hier Temperaturmodell, für die Magnetresonanzeinrichtung als Ganzes nachzuverfolgen. Dieser letztlich mittelnde Ansatz hat sich bereits als ausreichend erwiesen, um das Erreichen von Betriebsgrenzen erfolgreich nachvollziehen zu können.

Eine denkbare Weiterbildung der vorliegenden Erfindung kann auch vorsehen, dass bei einer benutzerseitigen Einstellbarkeit wenigstens eines Protokollparameters als weiterer Anpassungsparameter wenigstens ein die benutzerseitige Einstellbarkeit begrenzender Extremalwert wenigstens eines dieses wenigstens einen Protokollparameters ermittelt wird. Mit anderen Worten bedeutet diese, dass dann, wenn der Benutzer wünscht, Protokollparameter selber einzustellen, er ebenso einen Boostparameter, insbesondere unmittelbar die Wunschanzahl an Wiederholungen, vorgeben kann, so dass automatisch ermittelt werden kann, in welchen Wertebereichen sich die Protokollparameter bewegen dürfen, wobei hier auch Abhängigkeiten zu anderen Protokollparametern existieren können, die zu einer dynamischen Aktualisierung der bestimmten Extremalwerte führen können. Diese Ausgestaltung ist jedoch insgesamt weniger bevorzugt, da die vorliegende Erfindung auch auf eine bedienerisch möglichst einfach umsetzbaren Erweiterung der nutzbaren Leistungsfähigkeit der Magnetresonanzeinrichtung abzielt.

Neben dem Verfahren betrifft die Erfindung auch eine Magnetresonanzeinrichtung, die eine zur Durchführung des erfindungsgemäßen Verfahrens ausgebildete Steuereinrichtung mit der Benutzerschnittstelle, dem Controller und dem Prozessor aufweist. Sämtliche Ausführungen bezüglich des erfindungsgemäßen Verfahrens lassen sich analog auf die erfindungsgemäße Magnetresonanzeinrichtung übertragen, so dass auch mit dieser die bereits genannten Vorteile erhalten werden können.

Über die Benutzerschnittstelle kann die Steuereinrichtung, beispielsweise unter Nutzung eines GUI, Informationen von einem Benutzer entgegennehmen, insbesondere auch einen gewünschten Wert des Boostparameters sowie gegebenenfalls weitere Informationen, beispielsweise Wertebereiche für Anpassungsparameter, Lösungsstrategien und dergleichen, wie beschrieben wurde. Ein Prozessor bezeichnet im Rahmen der vorliegenden Erfindung allgemein eine Verarbeitungseinheit, die verschiedene funktionale Subeinheiten für unterschiedliche Schritte der vorliegenden Erfindung realisieren kann, im vorliegenden Fall wenigstens eine Ermittlungseinheit zur Ermittlung der Anpassungsparameter. Weitere, optionale Subeinheiten können beispielsweise eine Simulationseinheit umfassen, in der unter Benutzung des Zustandsparametermodells ein Ausrollen wenigstens des Protokollabschnitts erfolgen kann. Es können auch mehrere Prozessoren vorgesehen sein. Ein Prozessor kann beispielsweise eine CPU oder eine GPU sein.

Ein Controller ist im Rahmen der vorliegenden Erfindung als eine Steuereinheit zu verstehen. Vorliegend bildet der Controller wenigstens eine Sequenzeinheit, die mithin zur Aufnahme von Magnetresonanzdaten durch Ausspielen entsprechender Magnetresonanzsequenzen gemäß dem Protokoll und Empfang der entsprechenden Magnetresonanzsignale ausgebildet ist. Der Controller kann beispielsweise als wenigstens eine integrierte Schaltung und/oder wenigstens einen Chip umfassend realisiert werden. Die Steuereinrichtung kann ferner wenigstens ein Speichermittel umfassen.

Ein erfindungsgemäßes Computerprogramm ist beispielsweise direkt in einen Speicher einer Steuereinrichtung einer Magnetresonanzeinrichtung ladbar und weist Programmmittel auf, um die Schritte des erfindungsgemäßen Verfahrens auszuführen, wenn das Computerprogramm in der Steuereinrichtung der Magnetresonanzeinrichtung ausgeführt wird. Das Computerprogramm kann auf einem erfindungsgemäßen elektronisch lesbaren Datenträger gespeichert sein und umfasst mithin darauf gespeicherte elektronisch lesbare Steuerinformationen, welche zumindest ein genanntes Computerprogramm umfassen und derart ausgestaltet sind, dass sie bei Verwendung des Datenträgers in einer Steuereinrichtung einer Magnetresonanzeinrichtung das erfindungsgemäße Verfahren durchführen. Bei dem Datenträger handelt es sich insbesondere um einen nichttransienten Datenträger, beispielsweise eine CD-ROM.

Weitere Vorteile und Einzelheiten der vorliegenden Erfindung ergeben sie aus den im Folgenden beschriebenen Ausführungsbeispielen sowie anhand der Zeichnung. Dabei zeigen:
- Fig. 1: einen Ablaufplan eines Ausführungsbeispiels des erfindungsgemäßen Verfahrens,
- Fig. 2: eine mögliche Anzeige zur Umsetzung einer Benutzerschnittstelle,
- Fig. 3: ein mögliches Protokoll im zeitlichen Ablauf,
- Fig. 4: eine Prinzipskizze einer erfindungsgemäßen Magnetresonanzeinrichtung, und
- Fig. 5: den funktionalen Aufbau einer Steuereinrichtung der Magnetresonanzeinrichtung.

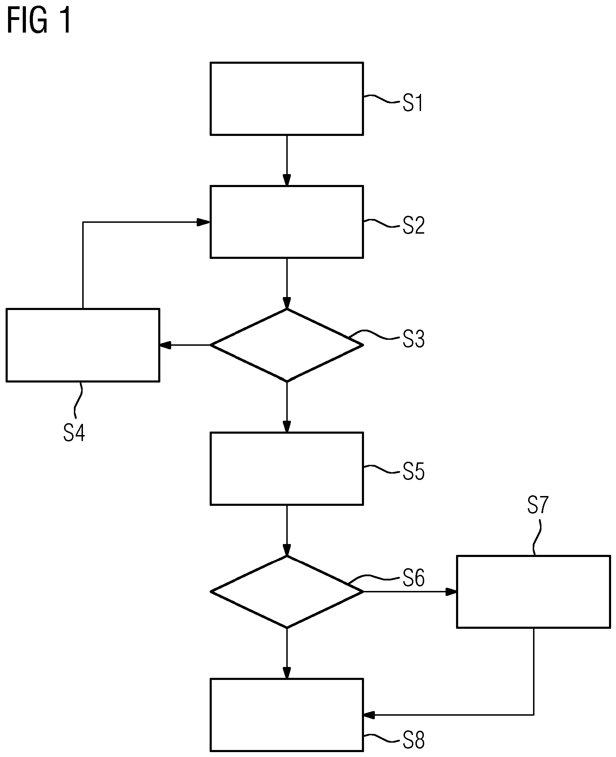

Fig. 1 zeigt einen Ablaufplan eines Ausführungsbeispiels des erfindungsgemäßen Verfahrens. Dabei wird vorliegend beispielhaft als Untersuchung mit einer Magnetresonanzeinrichtung eine Ganzkörper-Diffusionsmessung betrachtet, die durch ein Protokoll beschrieben wird, welches in einem Protokollabschnitt als Magnetresonanzsequenz eine EPI-Sequenz nutzt, die äußerst hohe Anforderungen an die Gradientenleistungsverstärker der Magnetresonanzeinrichtung stellt, so dass eine Erwärmung der Magnetresonanzeinrichtung bei der Durchführung dieses Protokollabschnitts auftreten kann.

Auf der anderen Seite enthält die Magnetresonanzeinrichtung Komponenten, die nicht überhitzt werden dürfen, so dass für die Temperatur der Magnetresonanzeinrichtung als Zustandsparameter eine Systemgrenze, beispielsweise in Form eines Schwellwertes, existiert, bei deren Überschreiten eine laufende Messung abgebrochen wird. Um ein derartiges Abbrechen im allgemeinen Fall zu vermeiden, ist in einer Steuereinrichtung der Magnetresonanzeinrichtung ein Protokollparametersatz abgelegt, der Protokollparameter für das Protokoll enthält, mit denen eine unbegrenzte Wiederholbarkeit des Protokolls, insbesondere also auch des Protokollabschnitts, sichergestellt ist, so dass dieser Protokollparametersatz mithin auch als RORE-Protokollparametersatz ("Run Once, Run Ever") bezeichnet werden kann.

Vorliegend ist es jedoch möglich, benutzerseitig gezielt über einen Boostparameter eine gegenüber dem RORE-Protokollparametersatz verbesserte Ausnutzung der Leistungsfähigkeit der Komponenten der Magnetresonanzeinrichtung zu erlauben, insbesondere, indem vorliegend die Messung beschleunigt wird, mithin Messpausen in dem Protokoll möglichst weitgehend vermieden werden, welche für eine Abkühlung vorgesehen sind. Ein entsprechender Boostparameter, der vorliegend direkt die Wunschanzahl von möglichen Wiederholungen des Protokollabschnitts beschreibt, mithin die maximal notwendig angenommenen Wiederholungen, kann über eine Benutzerschnittstelle eingegeben werden. Im RORE-Fall hat der Boostparameter den Wert ∞ (unendlich), so dass jeder endliche Wert für den Boostparameter höhere Anforderungen an die Hardware-Komponenten, hier insbesondere die Gradientenleistungsverstärkereinrichtung, erlaubt.

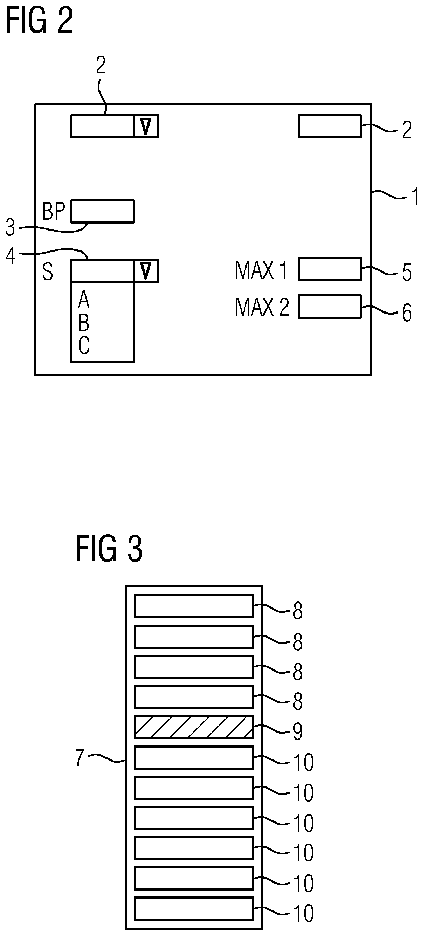

Der vom Benutzer gewünschte Wert des Boostparameters wird im Schritt S1 über die Benutzerschnittstelle entgegengenommen, mithin in einem Prozessor empfangen. Eine mögliche Anzeige 1 zur Realisierung eines GUI als Benutzerschnittstelle zeigt Fig. 2. Neben anderen Bedienelementen 2 ist in einem Eingabefeld 3 der Boostparameter, hier die Wunschanzahl an Wiederholungen (entspricht der angenommenen maximal notwendigen Anzahl an Wiederholungen) eingebbar.

Im nun folgenden Schritt S2 wird ein Teil der Protokollparameter des Protokolls, im Folgenden als Anpassungsparameter bezeichnet, neu ermittelt bzw. angepasst, insbesondere ausgehend von dem Protokollparametersatz. Der wenigstens eine Anpassungsparameter bezieht sich dabei insbesondere auf die EPI-Sequenz, mithin allgemein gesagt auf den Protokollabschnitt. Vorliegend ist im Übrigen ein Fall gegeben, in dem die restlichen Protokollanteile des Protokolls neben dem Protokollabschnitt die RORE-Anforderung erfüllen.

Im Schritt S2 wird nun von einem aktuellen Systemzustand der Magnetresonanzeinrichtung bezüglich des Zustandsparameters, hier der Temperatur, ausgegangen, um im Rahmen eines Optimierungsverfahrens für Testwerte des wenigstens einen Anpassungsparameters deren Auswirkung auf den Zustandsparameter, also die Temperatur, in der Magnetresonanzeinrichtung zu ermitteln. Mit anderen Worten wird der Protokollabschnitt mit der Wunschanzahl an Wiederholungen ausgerollt. Der aktuelle Systemzustand der Magnetresonanzeinrichtung, vorliegend also eine Art Starttemperatur zu Beginn des Protokollabschnitts, kann beispielsweise ohnehin ständig anhand eines Zustandsparametermodells mitgeführt werden, wird aber bevorzugt zumindest teilweise auch aus Messwerten von Temperatursensoren der Magnetresonanzeinrichtung bestimmt.

Der Bestimmung optimaler Anpassungsparameter liegt im vorliegenden Fall eine Lösungsstrategie zugrunde, die optional auch durch einen Benutzer vorgegeben werden kann. Hierzu ist gemäß Fig. 2 ein Dropdown-Menü 4 in der grafischen Benutzerschnittstelle vorgesehen, in welchem sich vorliegend drei Lösungsstrategien A, B und C auswählen lassen. Beispielsweise kann die Lösungsstrategie A für eine Minimierung der Echozeit TE, B für eine Minimierung der Repetitionszeit TR und C für eine Minimierung von TE und TR stehen. Insbesondere dann, wenn bezüglich zweier Anpassungsparameter, hier konkret der Echozeit TE und der Repetitionszeit TR optimiert werden soll, ist es ferner zweckmäßig, zum Ausschluss von ungeeigneten Insellösungen Maximalwerte für die Echozeit und die Repetitionszeit vorzugeben, was vorliegend für den Benutzer mittels der Eingabefelder 5 und 6 gemäß Fig. 2 möglich ist, welche aber auch automatisch, beispielsweise mit für den Anwendungsfall vorgegebenen Werten, ausgefüllt werden können.

Selbstverständlich sind auch andere Lösungsstrategien, insbesondere unter der Möglichkeit zur Anpassung weiterer Anpassungsparameter, denkbar, gegebenenfalls auch abstrakter vorgebbare Lösungsstrategien, beispielsweise Minimierung der Gesamtlaufzeit des Protokolls und Erhöhung der Bildqualität.

Im vorliegenden Fall wird zur Simulation der Effekte aktueller Testwerte für die Anpassungsparameter, mithin zum Ausrollen der Wiederholungen des Protokollabschnitts, ein einfaches Temperaturmodell als Zustandsparametermodell verwendet, welches eines allgemeine Temperaturangabe für die Magnetresonanzeinrichtung nutzt. Beispielsweise kann ein lineares Modell mit einem gleitenden Zeitfenster eingesetzt werden, welches eine mittlere Gradientenauslastung nutzt. Jedoch sind auch andere Möglichkeiten für Zustandsparametermodelle denkbar.

Es ist anzumerken, dass, wie bereits erwähnt, vorliegend davon ausgegangen wird, dass andere Protokollanteile neben dem Protokollabschnitt die RORE-Anforderung erfüllen, mithin die Temperatur als Zustandsparameter im Vergleich zum Protokollabschnitt nur unwesentlich beeinflussen, so dass es ausreichend ist, hier den Protokollabschnitt in seinen Wiederholungen auszurollen.

In einem Schritt S3 wird überprüft, ob das Optimierungsverfahren im Schritt S2 unter Berücksichtigung der Lösungsstrategie, der durch die Maximalwerte für die Echozeit und die Repetitionszeit vorgegebenen Wertebereiche und des Boostparameters eine Lösung auffinden konnte. Ist dies nicht der Fall, liegt ein Konflikt vor, der auf die vorgenommenen Einstellungen oder auf einen ungünstigen aktuellen Systemzustand zurückzuführen sein kann. Dann wird in einem Schritt S4 in einem Popup-Fenster der Benutzer angefragt, wie dieser Konflikt aufgelöst werden soll, beispielsweise durch eine Erhöhung des Boostparameters, durch Wahl einer anderen Lösungsstrategie oder durch Auswahl eines weniger optimal zu wählenden Anpassungsparameters. Danach wird im Schritt S2 erneut versucht, einen optimalen Anpassungsparametersatz zu finden.

In einem Schritt S5 wird dann der anhand des Anpassungsparametersatzes aktualisierte Protokollparametersatz genutzt, um Magnetresonanzdaten aufzunehmen. Mit anderen Worten wird das Protokoll unter Nutzung der bestimmten Werte für die Anpassungsparameter ausgespielt. Dabei wird, wie durch den Schritt S6 angedeutet, kontinuierlich überprüft, ob die Betriebsgrenzen eingehalten werden. Sollte die Temperatur beispielsweise im Schritt S6 trotz der vorgenommenen Berechnung im Schritt S2 die Grenztemperatur überschreiten oder sollte im Schritt S6 festgestellt werden, dass doch eine größere Anzahl an Wiederholungen als die Wunschanzahl erforderlich ist, wird in einem Schritt S7 der RORE-Protokollparametersatz als Rückfallebene genutzt, indem die Messung, trotz gegebenenfalls vorgenommenen Abbruchs aufgrund Erreichen der Betriebsgrenze, mit diesem RORE-Protokollparametersatz fortgesetzt wird. Denn dieser liefert äquivalente Magnetresonanzdaten, die die bereits unter Verwendung des Anpassungsparametersatzes aufgenommenen Magnetresonanzdaten des Protokolls ergänzen können und so die Messung effektiv vervollständigen können. Dabei steht eine erneute bzw. erstmalige Erreichung der Betriebsgrenze nicht zu befürchten, da die RORE-Protokollparameter gerade so gewählt sind, dass dies unter normalen Betriebsbedingungen nicht geschehen kann.

In jedem Fall wird in einem Schritt S8 das Ende des Verfahrens erreicht. Die aufgenommenen Magnetresonanzdaten können, wie üblich, nachbearbeitet und/oder gespeichert und/oder angezeigt werden.

Fig. 3 zeigt beispielhaft einen möglichen Ablauf eines Protokolls 7 zur Ganzkörper-Diffusionsbildgebung. In den zunächst ausgespielten Protokollanteilen 8 werden Localizer-Sequenzen verwendet, um verschiedene Localizerdaten unterschiedlicher Körperbereiche des Patienten aufzunehmen. Diese Localizer-Sequenzen stellen keine besonderen Anforderungen an die Leistungsfähigkeit der Magnetresonanzeinrichtung und sind daher grundsätzlich als RORE-Protokollanteile einzustufen.

Nach diesen erfolgt der Protokollabschnitt 9 mit der Wiederholung der EPI-Sequenzen zur eigentlichen Diffusionsmessung. Dieser Anteil ist schraffiert gezeigt, da er den die mit Abstand größte Leistungsanforderung stellenden Protokollanteil des Protokolls 7 bildet. Beispielsweise kann die Situation so sein, dass der Protokollabschnitt 9 selbst im RORE-Fall etwa das Zwanzigfache der Leistungsanforderung bezüglich der Gradientenleistungsverstärker im Vergleich zu den übrigen Protokollanteilen 8, 10 zur Folge hat.

Die folgenden Protokollanteile 10 betreffen weitere Aufnahmen mit anderen Sequenzen, beispielsweise MPRAGE-Sequenzen, VIBE-Sequenzen und dergleichen, die wiederum deutlich geringere Leistungsanforderungen stellen. Die dort aufgenommenen Magnetresonanzdaten können für interne Datenverarbeitungszwecke, beispielsweise Korrekturalgorithmen, dienen und/oder zur Erstellung von anatomischen Magnetresonanzbilddatensätzen, die beispielsweise zum Vergleich bzw. zur Verortung von Diffusionsphänomenen eingesetzt werden können.

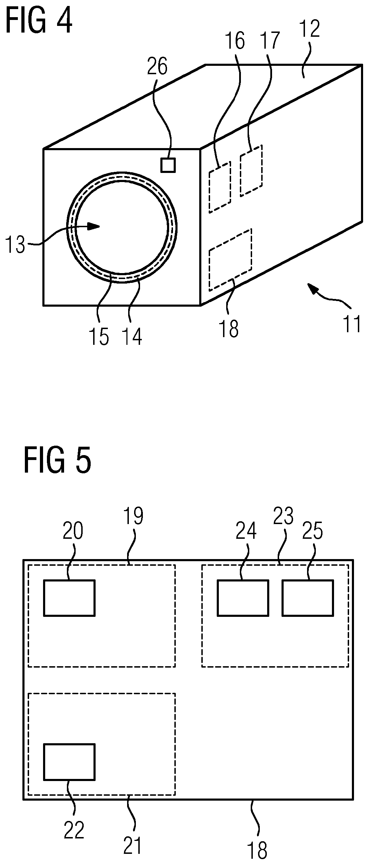

Fig. 4 zeigt eine Prinzipskizze einer erfindungsgemäßen Magnetresonanzeinrichtung 11. Diese umfasst, wie grundsätzlich bekannt, eine Hauptmagneteinheit 12 mit dem Grundfeldmagneten, in der eine Patientenaufnahme 13 definiert ist, in die ein Patient mittels einer hier nicht näher gezeigten Patientenliege eingefahren werden kann. Die Patientenaufnahme 13 umgebend sind vorliegend eine Gradientenspulenanordnung 14 und eine Hochfrequenzspulenanordnung 15 gezeigt. Entsprechend weist die Magnetresonanzeinrichtung 11 auch eine Gradientenleistungsverstärkereinrichtung 16 und eine Hochfrequenzleistungsverstärkereinrichtung 17 auf, welche sich auch wenigstens teilweise außerhalb der Schirmkabine, in der die Hauptmagneteinheit 12 befindlich ist, befinden können. Der Betrieb der Magnetresonanzeinrichtung 11 wird durch eine Steuereinrichtung 18 gesteuert. Über wenigstens einen Temperatursensor 26 kann zudem eine Temperatur der Magnetresonanzeinrichtung 11 erfasst werden.

Fig. 5 zeigt den funktionalen Aufbau der Steuereinrichtung 18 genauer. Diese weist zunächst einen Prozessor 19 auf, also eine Verarbeitungseinheit, wobei der Prozessor 19 vorliegend funktional wenigstens eine Ermittlungseinheit 20 zur Durchführung des Schrittes S2 und S3 realisiert, jedoch auch weitere Berechnungsfunktionen innerhalb der Steuereinrichtung 18 umsetzen kann. Die Interaktion mit einem Benutzer erfolgt über eine Benutzerinteraktionseinheit 21, die auch die Benutzerschnittstelle 22 umsetzt. Die Benutzerinteraktionseinheit 21 trägt also insbesondere zur Umsetzung der Schritte S1 und S4 bei.

Die Steuereinrichtung 18 umfasst ferner einen Controller 23, also eine Steuereinheit, wobei der Controller vorliegend wenigstens eine Sequenzeinheit 24 zur Durchführung des Schrittes S5 und des Schrittes S7 sowie eine Überwachungseinheit 25 zur Durchführung des Schrittes S6 implementiert.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung , der durch die beigefügten Ansprüche definiert ist, zu verlassen.

## Patentansprüche

1. Verfahren zum Betrieb einer Magnetresonanzeinrichtung (11), die wenigstens eine Komponente mit einer Betriebsgrenze bezüglich eines Zustandsparameters aufweist, zur Aufnahme von Magnetresonanzdaten mit einem wenigstens eine Magnetresonanzsequenz umfassenden Protokoll (7), wobei das Protokoll (7) durch Protokollparameter beschrieben ist und ein Protokollparametersatz vorliegt oder ermittelt wird, der bezüglich eines Protokollabschnitts (9) eine beliebig häufige Wiederholung des Protokollabschnitts (9) ohne Verletzung der Betriebsgrenze erlaubt, wobei das Verfahren folgende Schritte aufweist:
- Empfangen eines eine erhöhte Ausnutzung der Leistungsfähigkeit der Magnetresonanzeinrichtung (11) bei Begrenzung der Wiederholbarkeit des Protokollabschnitts (9) beschreibenden, benutzerseitig gewählten Boostparameters von einer Benutzerschnittstelle (22),
- zumindest für den Protokollabschnitt (9), wenigstens teilweise automatisches Ermitteln von Anpassungsparametern, die wenigstens einen Teil der Protokollparameter umfassen, unter Berücksichtigung des Boostparameters derart, dass eine durch den Boostparameter beschriebene Wunschanzahl an Wiederholungen bei Einhaltung der Betriebsgrenze gegeben ist, mittels eines Prozessors (19), und
- Aufnahme von Magnetresonanzdaten mit dem Protokoll (7) unter Verwendung der ermittelten Anpassungsparameter mittels eines Controllers (23),
wobei die Ermittlung der Anpassungsparameter unter Verwendung einer benutzerseitig wählbaren Lösungsstrategie erfolgt,
wobei die Lösungsstrategie eine Zielvorgabe beschreibt gewählt aus der Gruppe umfassend eine Reduzierung der Gesamtzeit zur Durchführung des Protokolls (7), Erhöhung der Magnetresonanzdatenqualität und Maximierung oder Minimierung wenigstens eines bestimmten Protokollparameters.

2. Verfahren nach Anspruch 1, **dadurch gekennzeichnet, dass** zur Ermittlung der Anpassungsparameter das Verhalten des wenigstens einen Zustandsparameters durch Ausrollen des Protokollabschnitts (9) oder unter Verwendung eines Zustandsparametermodells simuliert wird.

3. Verfahren nach Anspruch 1 oder 2, **dadurch gekennzeichnet, dass** bei einer durch einen Konflikt in Form eines nicht einhaltbaren, benutzerseitig vorgegebenen Wertebereichs für einen Anpassungsparameter gegebenen Nichtermittelbarkeit der Anpassungsparameter derart, dass die durch den Boostparameter beschriebene Wunschanzahl an Wiederholungen bei Einhaltung der Betriebsgrenze gegeben ist, als Konfliktstrategie wenigstens ein weiterer Protokollparameter oder der Boostparameter zur Lösung des Konflikts angepasst wird oder die Lösungsstrategie geändert wird.

4. Verfahren nach einem der vorangehenden Ansprüche, **dadurch gekennzeichnet, dass** bei der Ermittlung der Anpassungsparameter ein aktueller Systemzustand der Magnetresonanzeinrichtung (11) bezüglich des Zustandsparameters vor Beginn des Protokollabschnitts (9) berücksichtigt wird.

5. Verfahren nach einem der vorangehenden Ansprüche, **dadurch gekennzeichnet, dass** der Boostparameter unmittelbar die Wunschanzahl beschreibend empfangen wird.

6. Verfahren nach einem der vorangehenden Ansprüche, **dadurch gekennzeichnet, dass** bei einer benutzerseitig angeforderten oder automatisiert ausgelösten, die Wunschanzahl überschreitenden Anzahl von Wiederholungen des Protokollabschnitts (9) für die Wiederholungen nach der Wunschanzahl der Protokollparametersatz für unbegrenzte Wiederholungen verwendet wird oder eine Messpause eingefügt wird.

7. Verfahren nach einem der vorangehenden Ansprüche, **dadurch gekennzeichnet, dass** in außerhalb des Protokollabschnitts (9) gelegenen Protokollanteilen (8, 10) des Protokolls (7) lediglich beliebig oft wiederholbare Magnetresonanzsequenzen verwendet werden oder in dem Protokoll (7) mehrere Protokollabschnitte (9) definiert werden, denen jeweils Boostparameter zugeordnet werden.

8. Verfahren nach Anspruch 7, **dadurch gekennzeichnet, dass** bei mehreren Protokollabschnitten (9) der Einfluss des zeitlich früher durchgeführten Protokollabschnitts (9) bei der Wunschanzahl an Wiederholungen auf den Anfangssystemzustand der Magnetresonanzeinrichtung (11) bezüglich des Zustandsparameters bei Beginn des seitlich nächsten Protokollabschnitts (9) berücksichtigt wird.

9. Verfahren nach einem der vorangehenden Ansprüche, wobei die Magnetresonanzeinrichtungen eine Überwachungseinheit aufweist, und ausgelegt ist, wenn die Betriebsgrenze erreicht ist, einen Nothalt auszuführen und eine aktuell laufende Messung abzubrechen, **dadurch gekennzeichnet, dass** bei einem durch die Überwachungseinheit der Magnetresonanzeinrichtung (11) aufgrund der Überschreitung der Betriebsgrenze unterbrochenen Protokoll (7) dieses mit dem Protokollparametersatz für unbegrenzte Wiederholungen fortgesetzt oder neu gestartet wird.

10. Verfahren nach einem der vorangehenden Ansprüche, **dadurch gekennzeichnet, dass** der Zustandsparameter eine Temperatur der Komponente oder einen Ladezustand in einer Leistungsverstärkereinrichtung (16, 17) als Komponente beschreibt und/oder der Protokollabschnitt (9) eine Messung mit einer EPI-Magnetresonanzsequenz oder eine Diffusionsmessung oder eine Messung mit einer TSE-Sequenz umfasst.

11. Magnetresonanzeinrichtung (11), aufweisend eine zur Durchführung eines Verfahrens nach einem der vorangehenden Ansprüche ausgebildete Steuereinrichtung (18) mit der Benutzerschnittstelle (22), dem Controller (23) und dem Prozessor (19) .

12. Computerprogramm, welches die Schritte eines Verfahrens nach einem der Ansprüche 1 bis 10 durchführt, wenn es auf einer Steuereinrichtung (18) einer Magnetresonanzeinrichtung (11) ausgeführt wird.

13. Elektronisch lesbarer Datenträger, auf dem ein Computerprogramm nach Anspruch 12 gespeichert ist.

## Claims

1. Method for operating a magnetic resonance device (11) having at least one component that has an operating limit in relation to a state parameter, for recording magnetic resonance data using a protocol (7) that comprises at least one magnetic resonance sequence, wherein said protocol (7) is described by protocol parameters and a protocol parameter set is provided or determined which, in relation to a protocol section (9), allows said protocol section (9) to be repeated as often as desired without exceeding the operating limit, wherein the method has the following steps:
- receiving a boost parameter from a user interface (22), said boost parameter being selected by a user and describing an increased utilisation of the power output of the magnetic resonance device (11) while limiting the repeatability of the protocol section (9),
- at least for the protocol section (9), determining adaptation parameters in a manner which is at least partially automatic, said adaptation parameters comprising at least some of the protocol parameters, on the basis of the boost parameter in such a way that the desired number of repetitions described by the boost parameter is established while complying with the operating limit, by means of a processor (19), and
- recording magnetic resonance data with the protocol (7), using the determined adaptation parameters, by means of a controller (23),
wherein the determination of the adaptation parameters is effected using a solution strategy that is selectable by a user,
wherein the solution strategy describes an objective selected from the group comprising a reduction in the total time for performing the protocol (7), increasing the magnetic resonance data quality, and maximising or minimising at least one specific protocol parameter.

2. Method according to claim 1, **characterised in that** in order to determine the adaptation parameters, the response of the at least one state parameter is simulated by rolling out the protocol section (9) or using a state parameter model.

3. Method according to claim 1 or 2, **characterised in that** if the adaptation parameters cannot be determined due to a conflict in the form of a user-specified value range for an adaptation parameter that cannot be complied with, in order to establish the desired number of repetitions defined by the boost parameter while complying with the operating limit, as a conflict strategy, at least one further protocol parameter or the boost parameter is adapted in order to resolve the conflict and/or the solution strategy is changed.

4. Method according to one of the preceding claims, **characterised in that** when determining the adaptation parameters, a current system state of the magnetic resonance device (11) in relation to the state parameter before the start of the protocol section (9) is taken into consideration.

5. Method according to one of the preceding claims, **characterised in that** the received boost parameter directly describes the desired number.

6. Method according to one of the preceding claims, **characterised in that** if a number of repetitions of the protocol section (9) as requested by the user or automatically triggered exceeds the desired number, the protocol parameter set for unlimited repetitions is used or a measurement pause is inserted for the repetitions after to the desired number.

7. Method according to one of the preceding claims, **characterised in that** only magnetic resonance sequences which can be repeated as often as desired are used in protocol portions (8, 10) of the protocol (7) that are located outside of the protocol section (9) or a plurality of protocol sections (9) in the protocol (7) are defined which can be assigned a boost parameter in each case.

8. Method according to claim 7, **characterised in that** in the case of multiple protocol sections (9), the influence of the protocol section (9) which was performed previously, with the desired number of repetitions, on the initial system state of the magnetic resonance device (11) in relation to the state parameter, is taken into consideration at the start of the temporally subsequent protocol section (9).

9. Method according to one of the preceding claims, wherein the magnetic resonance devices has a monitoring unit and is configured, when the operating limit is reached, to perform an emergency stop and to terminate a measurement that is currently taking place, **characterised in that** if a protocol (7) is interrupted by the monitoring unit of the magnetic resonance device (11) due to an operating limit being exceeded, it is continued or restarted using the protocol parameter set for unlimited repetitions.

10. Method according to one of the preceding claims, **characterised in that** the state parameter describes a temperature of the component or a load state in a power amplifier device (16, 17) as a component, and/or the protocol section (9) comprises a measurement with an EPI magnetic resonance sequence and/or a diffusion measurement or a measurement with a TSE sequence.

11. Magnetic resonance device (11) with a control device (18) which is designed to perform a method according to one of the preceding claims and comprises the user interface (22), the controller (23) and the processor (19).

12. Computer program which performs the steps of a method according to one of the claims 1 to 10 when it is executed on a control device (18) of a magnetic resonance device (11).

13. Electronically readable data medium on which is stored a computer program according to claim 12.

## Revendications

1. Procédé pour faire fonctionner un dispositif (11) de résonnance magnétique, qui a au moins un composant ayant une limite de fonctionnement par rapport à un paramètre d'état, pour l'enregistrement de données de résonnance magnétique par un protocole (7) comprenant au moins une séquence de résonnance magnétique, dans lequel le protocole (7) est décrit par des paramètres de protocole et il y a ou on détermine un ensemble de paramètres de protocole, qui par rapport à une partie (9) du protocole permet une répétition fréquente à volonté de la partie (9) du protocole sans porter atteinte à la limite de fonctionnement, dans lequel le procédé a les stades suivants :
- réception par une interface (22) d'utilisateur d'un paramètre Boost choisi du côté de l'utilisateur et décrivant une utilisation augmentée de la capacité de puissance du dispositif (11) de résonnance magnétique à la limitation de la répétabilité de la partie (9) du protocole,
- au moins pour la partie (9) du protocole, détermination automatique, au moyen d'un processeur (19), au moins en partie de paramètres d'adaptation, qui comprennent au moins une partie des paramètres de protocole en tenant compte du paramètre Boost de manière à avoir, tout en conservant la limite de fonctionnement, un nombre souhaité, décrit par le paramètre Boost, de répétition, et
- l'enregistrement de données de résonnance magnétique par le protocole (7) en utilisant les paramètres d'adaptation déterminés au moyen d'une unité (23) de commande,
dans lequel la détermination des paramètres d'adaptation s'effectue en utilisant une stratégie de résolution pouvant être choisie du côté de l'utilisateur,
dans lequel la stratégie de résolution décrit une prescription de cible choisie dans le groupe comprenant une réduction de la totalité pour effectuer le protocole (7), une augmentation de la qualité des données de résonnance magnétique et une maximisation ou une minimisation d'au moins un paramètre déterminé du protocole.

2. Procédé suivant la revendication 1, **caractérisé en ce que** pour la détermination des paramètres d'adaptation, on simule le comportement du au moins un paramètre d'état en faisant se dérouler la partie (9) du protocole ou en utilisant un modèle de paramètre d'état.

3. Procédé suivant la revendication 1 ou 2, **caractérisé en ce que**, s'il y a une absence de possibilité de déterminer les paramètres d'adaptation donnée par un conflit sous la forme d'une plage de valeur d'un paramètre d'adaptation donnée à l'avance, du côté de l'utilisateur de manière à ce que le nombre souhaité, décrit par le paramètre Boost, de répétitions soit donné, tout en maintenant la limite de fonctionnement, on adapte comme stratégie de conflit au moins un autre paramètre du protocole ou le paramètre Boost pour résoudre le conflit ou on modifie la stratégie de résolution.

4. Procédé suivant l'une des revendications précédentes, **caractérisé en ce que**, lors de la détermination des paramètres d'adaptation, on prend en compte un état en cours du système du dispositif (11) de résonnance magnétique par rapport au paramètre d'état avant le début de la partie (9) du protocole.

5. Procédé suivant l'une des revendications précédentes, **caractérisé en ce qu'**on reçoit le paramètre Boost décrivant directement le nombre souhaité.

6. Procédé suivant l'une des revendications précédentes, **caractérisé en ce que** pour un nombre de répétitions de la partie (9) du protocole demandé du côté de l'utilisateur ou déclenché d'une manière automatisée, dépassant le nombre souhaité on utilise pour les répétitions, suivant le nombre souhaité, l'ensemble de paramètres du protocole pour des répétitions illimitées ou on insère une pause de mesure.

7. Procédé suivant l'une des revendications précédentes, **caractérisé en ce que** dans des parties (8, 10) du protocole (7) se trouvant à l'extérieur de la partie (9), on utilise simplement des séquences de résonnance magnétique répétables souvent à volonté ou on définit dans le protocole (7) plusieurs parties (9) du protocole, auxquelles on affecte respectivement des paramètres Boost.

8. Procédé suivant la revendication 7, **caractérisé en ce que** s'il y a plusieurs parties (9) du protocole, on prend en compte l'influence de la partie (9) du protocole effectuée antérieurement dans le temps lors du nombre souhaité de répétitions sur l'état initial du système du dispositif (11) de résonnance magnétique par rapport au paramètre d'état au début de la partie (9) du protocole suivante dans le temps.

9. Procédé suivant l'une des revendications précédentes, dans lequel le dispositif de résonnance magnétique a une unité de contrôle et est conçu pour, si la limite de fonctionnement est atteinte, rechercher un arrêt d'urgence et interrompre une mesure en cours de déroulement, **caractérisé en ce que** pour un protocole (7) interrompu par l'unité de contrôle du dispositif (11) de résonnance magnétique en raison du dépassement de la limite de fonctionnement, on poursuit ou on recommence celui-ci avec l'ensemble de paramètre du protocole pour des répétitions illimitées.

10. Procédé suivant l'une des revendications précédentes, **caractérisé en ce que** le paramètre d'état décrit une température du composant ou un état de charge dans un dispositif (16, 17) d'amplification de puissance comme composant et/ou la partie (9) du protocole comprend une mesure par une séquence de résonnance magnétique EPI ou une mesure de diffusion ou une mesure par une séquence TSE.

11. Dispositif (11) de résonnance magnétique comportant un dispositif (18) de commande constitué pour effectuer un procédé suivant l'une des revendications précédentes et ayant l'interface (22) d'utilisateur, l'unité (23) de commande et le processeur (19) .

12. Programme d'ordinateur, qui effectue les stades d'un procédé suivant l'une des revendications 1 à 10 lorsqu'il est exécuté sur un dispositif (18) de commande d'un dispositif (11) de résonnance magnétique.

13. Support de données, déchiffrable électroniquement, sur lequel un programme d'ordinateur suivant la revendication 12 est mis en mémoire.
